# EUROPEAN PATENT APPLICATION

(11) **EP 4 435 096 A1**
(43) Date of publication of application: **25.09.2024**
(21) Application number: 22895550.6
(22) Date of filing: 11.11.2022
(51) Int. Cl.: C12N 5/0793, C12N 5/071, C12N 5/10, C12N 15/12, C12N 15/861

(54) **CELL PRODUCTION METHOD**

(30) Priority: 17.11.2021 JP 2021186927
(71) Applicant: Takeda Pharmaceutical Company Limited, Osaka-shi, Osaka 541-0045 (JP)
(72) Inventor: IWATA, Hidehisa, Fujisawa-shi, Kanagawa 251-0012 (JP); NAGAI, Hiroaki, Fujisawa-shi, Kanagawa 251-0012 (JP); SAITO, Masayo, Fujisawa-shi, Kanagawa 251-0012 (JP)
(74) Representative: Jones, Nicholas Andrew
(86) International application number: PCT/JP2022/042056
(87) International publication number: WO 2023/090268

(57) **Abstract**

The present invention provides a method for inducing differentiation of motor neurons with less burden on a donor. The method for producing motor neurons from urine-derived cells according to the present invention includes a step of introducing into the urine-derived cell a transcription factor for inducing differentiation into a motor neuron. The transcription factor is introduced into the urine-derived cells by an adenoviral vector. The method of the present invention can further include a step of culturing the urine-derived cell into which the transcription factor has been introduced to obtain a cell positive for (I) at least one selected from the group consisting of Tuj1 and ISL1, and positive for (II) at least one selected from the group consisting of HB9, ChAT, and SMI32, preferably a cell positive for at least SMI 32 for (II).

## Description

### Technical Field

The present invention relates to a method for producing cells. More particularly, the present invention relates to a method for producing motor neurons by direct reprogramming.

### Background Art

A technique for directly obtaining target cells by introducing predetermined transcription factors into terminally differentiated somatic cells or the like (direct reprogramming), not via pluripotent stem cells such as iPS cells, is known; and as direct reprogramming in which target cells are motor neurons, the following methods are known.

Non Patent Literature 1 describes that HB9-positive motor neurons were obtained from human embryonic fibroblasts (HEF) derived from embryonic stem cells (ESC) by using 8 transcription factors (Asc1, Brn2, Myt1l, NEUROD1, Lhx3, Hb9, Isl1, Ngn2) and a retroviral vector.

Non Patent Literature 2 describes that motor neurons positive for Tuj1, ChAT and HB9 were obtained from human postnatal/adult fibroblasts by using 2 transcription factors (NGN2 and Sox11) and a lentiviral vector. On the other hand, Non Patent Literature 2 also describes that the expression of ISL1 and LHX3 was not observed.

Non Patent Literature 3 describes that motor neurons positive for Tuj1, HB9, ChAT and the like were obtained from human adult fibroblasts by using 4 transcription factors (NGN2, Sox11, ISL1 and LHX3) and a lentiviral vector.

Non Patent Literature 4 describes that motor neurons positive for Tuj1, HB9, ChAT and the like were obtained from human adult fibroblasts by using 4 transcription factors (NGN2, Sox11, ISL1 and LHX3) and a lentivirus.

Non Patent Literature 5 describes that motor neurons positive for Tuj1, HB9, ChAT, SMI32 and the like were obtained from human adult fibroblasts by using 2 transcription factors (ISL1 and LHX3) as well as 2 miRNAs (miR-9/9* and miR-124).

Non Patent Literature 6 describes that neurons positive for Tuj1 were obtained from mouse fibroblasts by using 3 transcription factors (Ascl1, Brn2 and Myt1l or Ascl1, Brn2 and Ngn2) and an adenoviral vector (however, the expression of motor neuron markers was not observed, nor was the neuron type identified.).

However, Non Patent Literatures 1 to 6 are all reports on direct reprogramming from fibroblasts to motor neurons, and do not describe that motor neurons were obtained from urine-derived cells by direct reprogramming.

On the other hand, Non Patent Literature 7 describes that there are some reports on the fact that nerve cells are obtained from urine-derived cells by direct reprogramming, but do not describe that motor neurons were obtained.

### Citation List

### Non Patent Literature

Non Patent Literature 1: Esther Y. Son et al. (2011) Cell Stem Cell 9, 205-218
Non Patent Literature 2: Meng-Lu Liu et al. (2013) Nature Communications, DOI:10.1038
Non Patent Literature 3: Meng-Lu Liu et al. (2016) Cell Reports 14, 115-128
Non Patent Literature 4: Yu Tang et al. (2017) Frontiers in Molecular Neuroscience, Vol. 10, Article 359
Non Patent Literature 5: Daniel G. Abernathy, st al. (2017) Cell Stem Cell 21, 332-348
Non Patent Document 6: Cell Research (2012) 22:436-440
Non Patent Literature 7: Mitsuo Sato et al. (2019) Frontiers in Molecular Neuroscience, Vol. 12, Article 297

### Summary of Invention

### Technical Problem

As a conventional method for inducing differentiation of motor neurons, direct reprogramming from fibroblasts as described above has generally been used; however, punching when collecting fibroblasts was highly invasive and the burden on donors was large.

The present invention aims to provide a method for inducing differentiation of motor neurons with less burden on a donor.

### Solution to Problem

The present inventors have found that urine-derived cells, which can be noninvasively collected from urine of a donor, can be used to induce differentiation into motor neurons, by direct reprogramming that introduces into the cells a transcription factor for inducing differentiation into motor neurons. In addition, according to this method, a cell population including motor neurons that have a high positive rate of cells expressing motor neuron markers, the length of nerve fibers (degrees of differentiation and maturity of motor neurons) extending excellently, and are functional to skeletal muscle cells, compared to conventional direct reprogramming from fibroblasts.

In other words, the present invention includes at least the following matters.
[1] A method for producing a motor neuron from a urine-derived cell, including
   a step of introducing into the urine-derived cell a transcription factor for inducing differentiation into a motor neuron.
[2] The method according to [1], wherein the transcription factor is introduced into the urine-derived cell by an adenoviral vector.
[3] The method according to [1] or [2], further including a step of culturing the urine-derived cell into which the transcription factor has been introduced to obtain a cell positive for (X) at least one selected from the group consisting of ISL1, LHK3, HB9, ChAT, SMI32, and VAChT.
[4] The method according to [1] or [2], further including a step of culturing the urine-derived cell into which the transcription factor has been introduced to obtain a cell positive for (X) at least one selected from the group consisting of ISL1, LHK3, HB9, ChAT, SMI32, and VAChT, and positive for (Y) at least one selected from the group consisting of Tuj1, MAP2, NeuN, neurofilament, synapsin, and PSD-95.
[5] The method according to [1] or [2], further including a step of culturing the urine-derived cell into which the transcription factor has been introduced to obtain a cell positive for (I) at least one selected from the group consisting of Tuj1 and ISL1, and positive for (II) at least one selected from the group consisting of HB9, ChAT, and SMI32.
[6] The method according to one of [3] to [5], wherein the step is to obtain a cell positive for at least SMI32.
[6a] The method according to one of [1] to [6], wherein the transcription factor contains a neurodifferentiation factor and a motor neuron differentiation factor.
[7] The method according to one of [1] to [6a], wherein the transcription factor contains (i) at least one neurodifferentiation factor selected from the group consisting of NGN2, ASCL1, BRN2, MYT1L, NEUROD1, and miR-9/9*-124, and (ii) at least one motor neuron differentiation factor selected from the group consisting of ISL1, LHX3, and HB9.
[7a] The method according to one of [1] to [7], wherein the transcription factor further contains SOX11.
[8] The method according to one of [1] to [7a], wherein the urine-derived cell into which the transcription factor has been introduced is cultured in a medium containing one selected from the group consisting of a basic fibroblast growth factor, forskolin, and dorsomorphin.
[9] A cell population containing a motor neuron obtained by the method according to one of [1] to [8].

### Advantageous Effects of Invention

According to the present invention, differentiation into motor neurons can be induced without burden on a donor, by using urine-derived cells obtained by the donor, and also there is a tendency to have a high positive rate of predetermined markers for motor neurons, to be excellent in the extension of the length of nerve fibers, and to be functional to skeletal muscle cells, compared to the induction of differentiation using fibroblasts, which was conventionally common. For example, when using urine-derived cells obtained from a patient with a motor neuron-related disease (e.g., amyotrophic lateral sclerosis, ALS), since the motor neurons induced to differentiate from the urine-derived cells also contain genetic information unique to the patient, the motor neurons can be used to accurately perform drug evaluation, biomarker search and the like for ALS treatment of the patient.

### Brief Description of Drawings

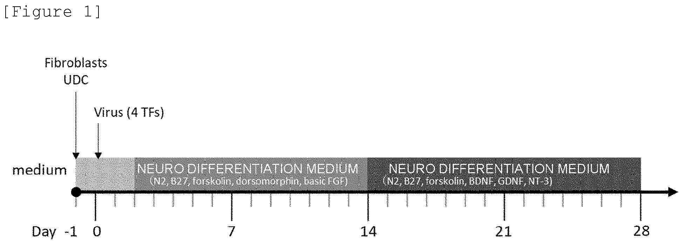

[Figure 1] A figure illustrating differentiation of motoneurons induced by a lentivirus and an adenoviral vector. Illustrated is an experimental procedure for preparing motoneurons (MN) from human fibroblasts or UDCs by introducing 4 transcription factors (4 TFs) of hNGN2, mSox11, hISL1 and hLHX3.

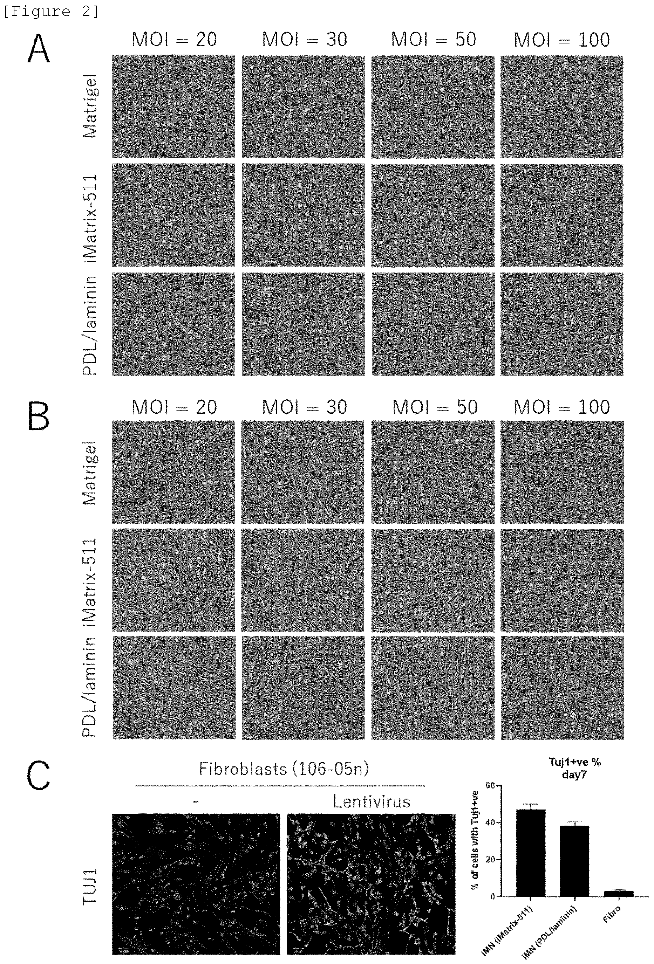

[Figure 2] Figures for comparing coating agents with MOI when motoneurons are induced to differentiate from human fibroblasts by a lentivirus. According to the procedure shown in Figure 1, human fibroblasts (106-05n) were infected with 2 lentiviruses and induced to differentiate into motoneurons to obtain bright field images on (Figure 2A) day 7 and (Figure 2B) day 14. Scale bar = 50 µm. Figure 2C shows immunofluorescent staining images for Tuj1 (neuron marker) on days 0 and 7 of differentiation induction, and quantitative results of expression rates. Scale bar = 50 µm. Error bars represent the standard deviation (SD), and N = 3.

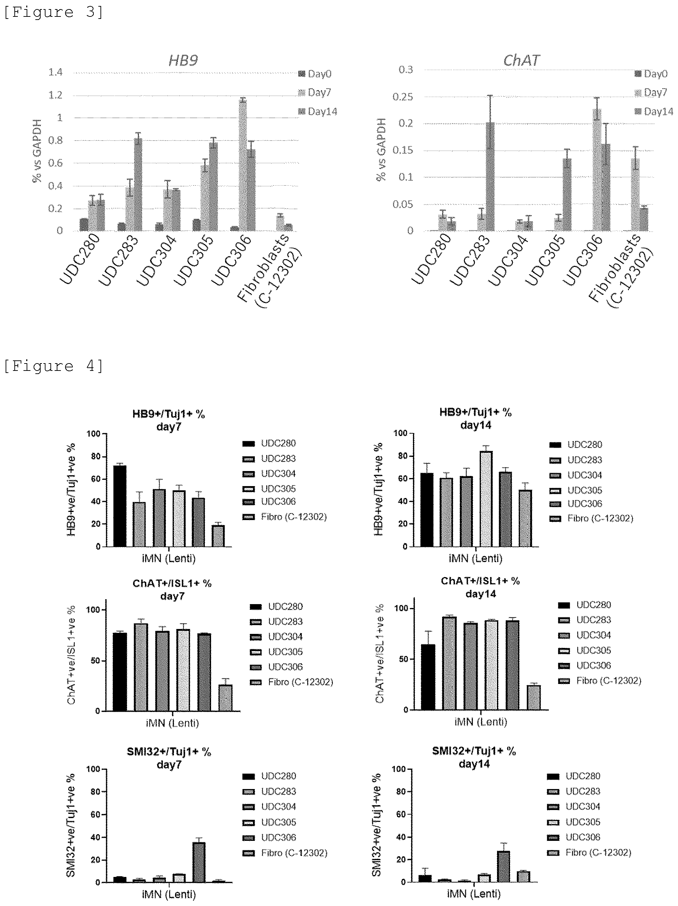

[Figure 3] Figures illustrating gene expression of motoneuron markers in motoneurons induced to differentiate from human UDCs by a lentivirus. According to the procedure shown in Figure 1, human UDCs were infected with 2 lentiviruses and induced to differentiate into motoneurons. Human fibroblasts (C-12302) were used as a comparison. Gene expression analysis of motoneuron markers (HB9 and ChAT) on days 0, 7 and 14 of differentiation induction by qPCR. Error bars represent SD, and N = 3.
[Figure 4] Figures illustrating expression of neuron markers and motoneuron markers in motoneurons induced to differentiate from human UDCs by a lentivirus. According to the procedure shown in Figure 1, human UDCs were infected with 2 lentiviruses and induced to differentiate into motoneurons. Human fibroblasts (C-12302) were used as a comparison. Quantitative results of each marker expression rate on days 0, 7 and 14 of differentiation induction are shown.

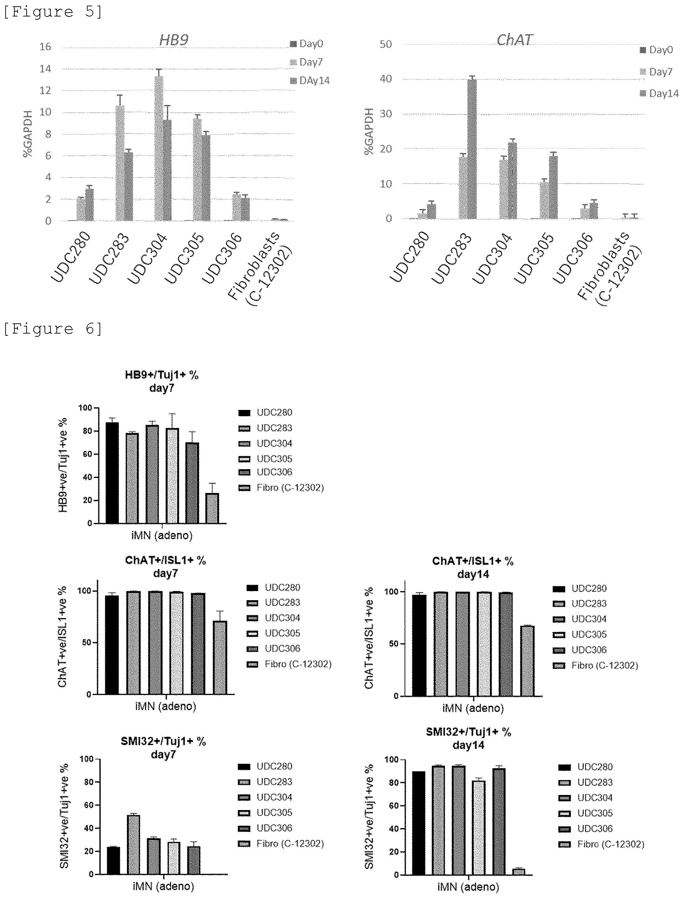

[Figure 5] Figures illustrating expression of neuron markers and motoneuron markers in motoneurons induced to differentiate from human UDCs by an adenovirus. According to the procedure shown in Figure 1, human UDCs were infected with 2 adenoviruses and induced to differentiate into motoneurons. Human fibroblasts (C-12302) were used as a comparison. Gene expression analysis of motoneuron markers (HB9 and ChAT) on days 0, 7 and 14 of differentiation induction by qPCR. Error bars represent SD, and N = 3.
[Figure 6] Figures illustrating expression of neuron markers and motoneuron markers in motoneurons induced to differentiate from human UDCs by an adenovirus. According to the procedure shown in Figure 1, human UDCs were infected with 2 adenoviruses and induced to differentiate into motoneurons. Human fibroblasts (C-12302) were used as a comparison. Quantitative results of each marker expression rate on days 0, 7 and 14 of differentiation induction are shown.

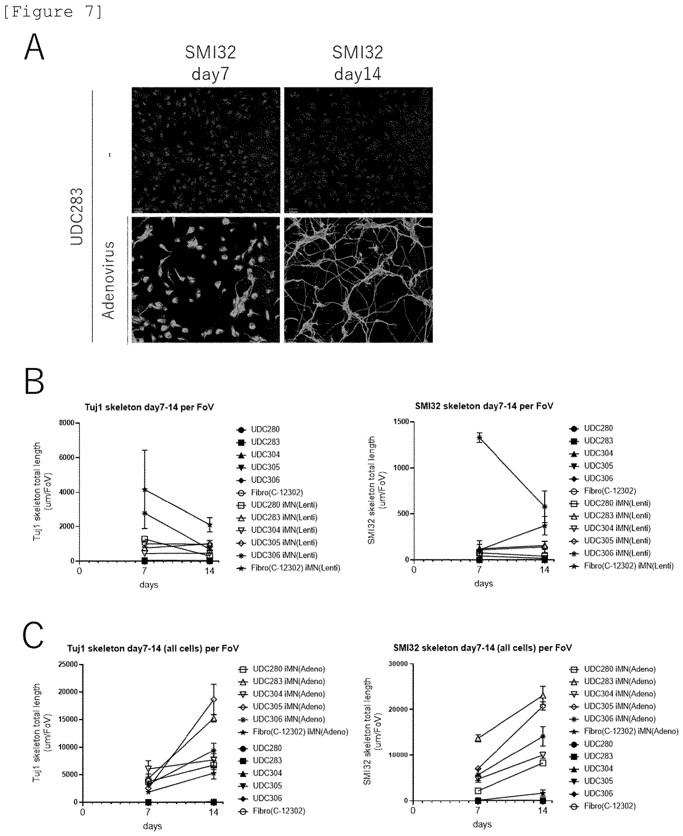

[Figure 7] Figures illustrating the length of nerve fibers of a neuron marker TUJ1 and a motoneuron marker SMI32 in motoneurons induced to differentiate from human UDCs by a lentivirus or an adenovirus. According to the procedure shown in Figure 1, human UDCs were infected with 2 lentiviruses or 2 adenoviruses and induced to differentiate into motoneurons. Human fibroblasts (C-12302) were used as a comparison. Figure 7A shows immunofluorescent staining images for SMI32 on days 0, 7 and 14 of differentiation induction by an adenovirus. Scale bar = 50 µm. Figure 7B shows quantitative results of the length of nerve fibers of TUJ1 and SMI32 on days 0, 7 and 14 of differentiation induction by a lentivirus. Figure 7C shows quantitative results of the length of nerve fibers of TUJ1 and SMI32 on days 0, 7 and 14 of differentiation induction by an adenovirus.

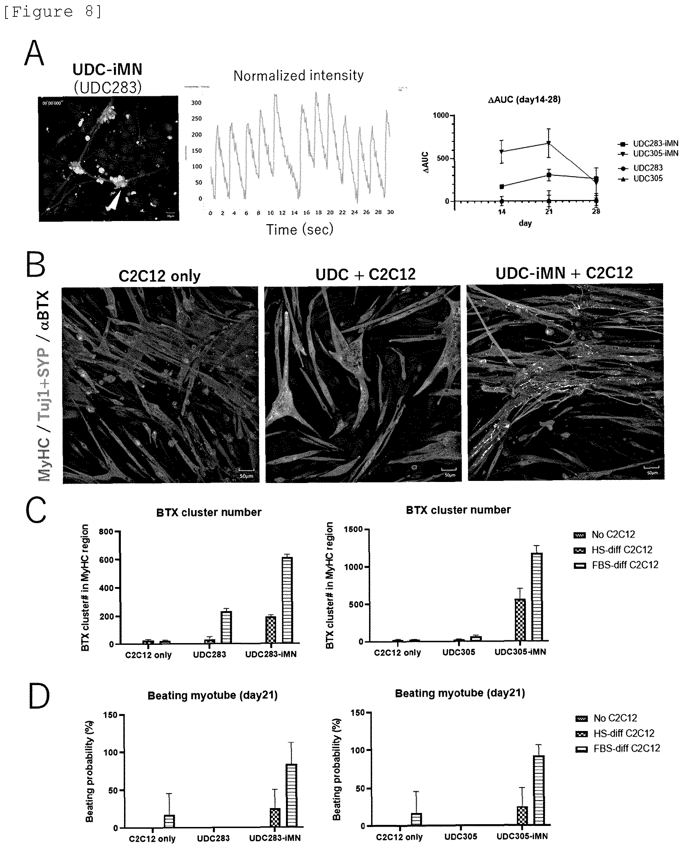

[Figure 8] Figures illustrating the functionality of motoneurons induced to differentiate from human UDCs by an adenovirus. According to the procedure shown in Figure 1, human UDCs were infected with 2 adenoviruses and induced to differentiate into motoneurons. Figure 8A shows results of calcium imaging on days 14, 21 and 28 of differentiation induction by an adenovirus. Scale bar = 30 µm. Figure 8B shows immunofluorescent staining images on the formation of neuromuscular junction (NMJ) when motoneurons 14 days after differentiation induction by UDCs or adenoviruses and skeletal muscle cells were co-cultured for 7 days. Scale bar = 50 µm. Figure 8C shows quantitative results of the number of positive clusters for α-bungarotoxin on the formation of NMJ when motoneurons 14 days after differentiation induction by UDCs or adenoviruses and skeletal muscle cells were co-cultured for 7 days. Figure 8D shows results of contraction probabilities of skeletal muscle cells when motoneurons 14 days after differentiation induction by UDCs or adenoviruses and the skeletal muscle cells were co-cultured for 7 days.

### Description of Embodiments

The term "marker" used herein means a "marker protein" or a "marker gene" that is a protein or a gene thereof specifically expressed on a cell surface, in cytoplasm, in a nucleus and/or the like in a predetermined cell type. The marker may be a positive selection marker or negative selection marker. Preferably, the marker is a cell surface marker, and in particular, using a cell surface positive selection marker allows enrichment, isolation, and/or detection of live cells to be performed.

Detection of the marker protein can be performed by an immunological assay using an antibody specific to the marker protein, for example, ELISA, immunostaining, or flow cytometry. As the antibody specific to the marker protein, an antibody to be bound to a specific sugar chain that has been bound to a specific amino acid sequence in the marker protein or the marker protein can be used. In addition, it is possible to use a technique in which a marker protein (e.g., transcription factor or a subunit thereof, cytokine) that is expressed in a cell but not on a cell surface (on a cell membrane), or that is secreted from a cell is used as a target, the cell is fixed thereon, and then the marker protein is fluorescent-stained in the cell using an antibody specific to the marker protein, or a reporter protein is made to be expressed with the marker protein. The technique is preferably used when no suitable cell surface marker is found. On the other hand, detection of a marker gene can be performed by a nucleic acid amplification method and/or a nucleic acid detection method known in the art, such as RT-PCR (including quantitative PCR), a microarray, a biochip, and RNAseq.

As used herein, the term "positive" for a marker or the like means the expression level of a protein or gene such as a marker (measurement value or signal reflecting the level) exceeds (or is equal to or more than) a detectable amount by a method known in the art as described above or a predetermined reference value. As used herein, the term "negative" for a marker or the like means the expression level of a protein or gene such as a marker is less than (or equal to or less than) a detectable amount by all or any of the methods known in the art as described above or a predetermined reference value. The detectable amounts or reference values of a protein or gene expressed may vary depending on the employed technique or the purpose of the analysis. For example, in a case of the expression level (amount to be secreted) of a protein serving as a marker, it can be determined to be "positive" when a fluorescence signal on a flow cytometry that stains a cell with a fluorescence labeling antibody (representative example thereof is fluorescence activated cell sorting, FACS) is higher than (equal to or higher than) a predetermined reference set based on a fluorescence signal of a non-stained signal, and to be "negative" when the signal is lower (equal to or lower). When the expression is positive, it may be indicated as a symbol "+", when negative, it may be indicated as a symbol "-". As used herein, the "positive rate" or "negative rate" of a predetermined marker or the like in a cell population means a rate of cells in which the predetermined marker or the like is "positive" or "negative" to a certain number of cells contained in the cell population. The "positive rate" or "negative rate" can be measured according to a conventional method, for example, by using a fluorescent immunostaining image, or by using flow cytometry (FACS).

The method for producing a motor neuron from a urine-derived cell of the present invention (also referred herein to as "production method of the present invention") includes a step of introducing into the urine-derived cell a transcription factor for inducing differentiation into a motor neuron.

The "urine-derived cells" (UDCs) are a mixture of various cells derived from urinary system tissues, such as kidney, ureter, bladder, and urethra, contained in the urine of a subject (donor). The urine-derived cells can be separated and collected from urine according to a conventional method (e.g., by centrifugation). In the present invention, the urine-derived cells thus collected can be used, or cultured cell lines obtained by establishing the collected urine-derived cells, for example, commercially available products, can be used. The urine-derived cells may be derived from a human, or a non-human animal, for example, a mammal such as mouse, rat, dog, pig or monkey, which can be selected according to the usage of motor neurons obtained in the present invention. For example, when using urine-derived cells collected from urine of a patient with amyotrophic lateral sclerosis (ALS) to induce differentiation into motor neurons by the production method of the present invention, the motor neurons will be suitable for drug evaluation, biomarker search and the like for ALS treatment of the patient.

Noted that the urine-derived cells may also be referred to as "urine-derived stem cells", "urine-derived progenitor cells", "cells voided in urine" and the like, and those skilled in the art can understand these cells refer to cells of the same concept.

The "motor neurons" (also written as "motoneurons" in Japanese) are nerve cells that control skeletal muscles and include upper motor neurons in the motor cerebral cortex and lower motoneurons in the brainstem and the spinal cord. Degeneration of upper motor neuron and/or lower motor neuron causes motor neuron diseases such as amyotrophic lateral sclerosis, primary lateral sclerosis, progressive pseudobulbar palsy, progressive muscular atrophy, progressive bulbar palsy, and post-polio syndrome. Whether or not a cell is a motor neuron can be determined by whether it is positive (or negative) for a predetermined marker as described below.

In one embodiment of the present invention, a motor neuron obtained by inducing differentiation from a urine-derived cell according to the production method of the present invention is a cell positive for at least one motor neuron marker (also referred herein to as "MN marker") (e.g., ISL1, LHX3, HB9, ChAT, SMI32, and VAChT). In one preferred embodiment of the present invention, a motor neuron obtained by inducing differentiation from a urine-derived cell according to the production method of the present invention is a cell positive for at least one neuronal marker (e.g., Tuj1, MAP2, NeuN, neurofilament, synapsin, and PSD-95) and positive for at least one MN marker. In one particularly preferred embodiment of the present invention, a motor neuron obtained by inducing differentiation from a urine-derived cell according to the production method of the present invention is a cell positive for at least one neuronal marker selected from the group consisting of Tuj1 and ISL1 and positive for at least one MN marker selected from the group consisting of HB9, ChAT and SMI32.

In one preferred embodiment of the present invention, a motor neuron obtained by inducing differentiation from a urine-derived cell according to the production method of the present invention is positive for at least SMI32 among the above predetermined markers.

The "transcription factor for inducing differentiation into a motor neuron" used herein (also referred to as "transcription factor for MN") is a generic term for a "neurodifferentiation factor" that is a factor for inducing differentiation of a cell into a nerve cell and a "motor neuron differentiation factor" that is a factor for inducing differentiation of a nerve cell into a motor neuron, as well as an "additional factor" that is an auxiliary factor to be used as necessary (e.g., for improving conversion efficiency to a nerve cell). Such transcription factors for MN are known, and for example, NGN2, ASCL1, BRN2, MYT1L, NEUROD1, and miRNAs such as miR-9/9* and miR-124 correspond to the "neurodifferentiation factors", ISL1, LHX3, HB9 and the like correspond to the "motor neuron differentiation factors", and SOX11 corresponds to the "additional factor", which are described in Non-Patent Literatures 1 to 6 above. The transcription factors for MN may be homologs of the above genes (proteins) in non-human animal species (e.g., mouse). Homologs of transcription factors for MN can be searched on databases such as DNA Data Bank of Japan (DDBJ), NCBI GenBank, and EMBL. The transcription factors for MN are usually used by combining multiple types thereof, and those skilled in the art can select an appropriate combination of transcription factors for MN.

In one embodiment of the present invention, transcription factors for MN contains at least a neurodifferentiation factor and a motor neuron differentiation factor. In one preferred embodiment of the present invention, transcription factors for MN contains a neurodifferentiation factor, a motor neuron differentiation factor and an additional factor.

In one preferred embodiment of the present invention, the transcription factor for MN contains (i) at least one neurodifferentiation factor selected from the group consisting of NGN2, ASCL1, BRN2, MYT1L, NEUROD1, and miR-9/9*-124, and (ii) at least one motor neuron differentiation factor selected from the group consisting of ISL1, LHX3, and HB9, and may further contain (iii) SOX11 as an additional factor. In one preferred embodiment of the present invention, the transcription factor for MN contains (i) NGN2 that is a neurodifferentiation factor, and (ii) at least one motor neuron differentiation factor selected from the group consisting of ISL1 and LHX3, and may further contain (iii) SOX11 as an additional factor. In one further preferred embodiment of the present invention, the transcription factor for MN contains (i) NGN2 as a neurodifferentiation factor, (ii) both ISL1 and LHX3 as motor neuron differentiation factors and (iii) SOX11 as an additional factor.

The transcription factor for MN can be introduced into the urine-derived cell in the form of a gene (nucleic acid) or in the form of a protein that is a product of the gene. Means for introducing genes (nucleic acids) or proteins into cells are known to those skilled in the art, and appropriate means and corresponding conditions can be used in the present invention. The gene (nucleic acid) of the transcription factor for MN may be, for example, in the form of DNA such as a plasmid or an expression vector, or in the form of RNA such as mRNA, and for example, can be introduced into the urine-derived cell by a known method such as a viral infection method, a calcium phosphate method, a lipofection method, a microinjection method, or an electroporation method. In addition, the protein of the transcription factor for MN can be introduced into the urine-derived cell by, for example, coupling with cell-penetrating peptides.

For example, the transcription factor for MN can be introduced into the urine-derived cell in the form of an expression vector (viral vector plasmid, expression plasmid, etc.) into which a gene for encoding the transcription factor for MN is inserted. The expression vector may be double stranded or single-stranded, and may be DNA or RNA. The expression vector can be in an embodiment in which the vector resides in a nucleus or cytoplasm temporarily or persistently while being replicated, or in an embodiment in which the vector resides permanently while being incorporated into a genome DNA. Multiple types of the transcription factors for MN can be expressed using one expression vector including all types, or can be expressed in such a way that one expression vector includes one or a part of the types of the transcription factors for MN and a plurality of such expression vectors are combined to be used for expression. Expressing all genes of predetermined transcription factors for MN in the urine-derived cells leads to produce proteins of the transcription factors for MN, and as a direct or indirect result, the urine-derived cells are induced to differentiate into motor neurons.

The transcription factor for MN is preferably introduced into the urine-derived cell in the form of an expression vector by a viral infection method. For example, the following method can be mentioned: using commercially available kits that address each of retroviral vector, lentiviral vector, adenoviral vector, and adeno-associated viral vector, transfecting host cells with an expression vector and packaging vectors of each virus (plasmid) to prepare a recombinant virus followed by infecting urine-derived cell with the thus obtained recombinant virus.

In one embodiment of the present invention, the transcription factor for MN is introduced into the urine-derived cell by an adenoviral vector. Use of an adenoviral vector is preferable from the viewpoint that, for example, the positive rate of a predetermined MN marker is higher (e.g., higher than a case of using a lentiviral vector), a cell population of motor neurons that are morphologically maturated such as the neurite length can be obtained, and the difference of the positive rates of the predetermined MN markers of motor neurons among the donors of the urine-derived cells are small and all of the positive rates tend to be high.

The production method of the present invention includes, a step of introducing a transcription factor and then culturing the urine-derived cell into which the transcription factor has been introduced to obtain a cell (i.e., motor neuron) positive for a predetermined marker as described above (also referred herein to as "differentiation induction step").

The culturing period in the differentiation induction step can be, for example, 1 day or more, preferably 1 week or more, more preferably 2 weeks or more, and further preferably 3 weeks or more. The upper limit of the culturing period in the differentiation induction step is not particularly limited. Specifically, it can be, for example, 1 to 12 weeks, preferably 1 to 8 weeks, more preferably 1 to 4 weeks, further preferably 1 to 3 weeks, and particularly preferably 2 to 3 weeks. In the differentiation induction step, the culturing period can be appropriately adjusted within the range as above depending on urine-derived cells and transcription factors for MN to be used, and culturing conditions such as culture medium or the like, in order to obtain a cell population that has a desired cell composition (e.g., having the expression profile and the positive rate of a predetermined marker).

In the present invention, the medium for inducing differentiation of urine-derived cells into motor neurons can be appropriately selected from various known media and used by adding components as necessary at an appropriate concentration. The basal medium and components to be added can be changed depending on the steps of the production method of the present invention (transcription factor introduction step, differentiation induction step, or the like) or along with the passage of the culturing days. For example, as the medium in the transcription factor introduction step, a mixture of a basal medium for renal epithelial cells and components to be added can be used; and as the medium in the differentiation induction step, a mixture of a basal medium for nerve cells and components to be added can be used.

Examples of the basal medium include AIM V, X-VIVO-15, NeuroBasal, EGM2, TeSR, BME, BGJb, CMRL 1066, Glasgow's MEM, improved MEM zinc option, IMDM, 199 medium, Eagle's MEM, αMEM, DMEM, Ham, RPMI-1640, F12, and Fisher's medium. Any one of these media can be used alone, or two or more can be mixed for use. An example of a preferable medium include (high glucose) DMEM, F12. In addition, in order to prepare a medium for urine-derived cells in the transcription factor introduction step (as a mixed medium with other basal media), for example, Renal Epithelial Cell Growth Medium Bullet Kit (REGM, Lonza Group Ltd.) can be used; and in order to prepare a medium for nerve cells in the differentiation induction step (as a mixed medium with other basal media), for example, Neurobasal Medium (GIBCO) can be used.

The medium may be a medium containing serum (e.g., fetal bovine serum: FBS) or a medium containing no serum (serum-free medium: SFM), and may also be a xeno-free medium. From the viewpoint of inhibiting contamination due to xenogenic animal-derived components, serum can be derived from the same animal as that from which cells are cultured. The serum-free medium refers to a medium without containing unprocessed or unpurified serum, and therefore, it may include purified blood-derived components or animal tissue-derived components (e.g., growth factor). The medium may or may not contain any replacements for serum. Examples of the serum replacements may include materials appropriately containing albumin (albumin substitutes such as lipid-rich albumin, bovine albumin, recombinant albumin, or humanized albumin, plant starch, dextran, and protein hydrolysate), transferrin (or other iron transporters), fatty acids, insulin, collagen precursors, microelements, 2-mercaptoethanol, 3'-thioglycerol (α-monothioglycerol, MTG), or equivalents thereof. Commercially available materials such as knockout serum replacements (KSR), chemically-defined lipid concentrated (Gibco), and GlutaMAX (Gibco) can also be used. Examples of the medium may include B-27 (registered trademark) supplements, xeno-free B-27 (registered trademark) supplements, N2 supplements, NS21 supplements, GS21 (trademark) supplements, or a combination thereof.

The medium can contain one or two or more selected from the group consisting of biotin; DL-alpha-tocopherol acetate; DL-alpha-tocopherol; vitamins such as vitamin A (acetate); BSA (bovine serum albumin) or human albumin, fatty acid free fraction V; catalase; human recombinant insulin; human transferrin; proteins such as superoxide dismutase; corticosterone; D-galactose; ethanol amine HCl; glutathione (reduced form); L-carnitine HCl; linoleic acid; linolenic acid; progesterone; putrescine 2HCl; sodium selenite; and T3 (triiodo-I-thyronine); PSG (penicillin, streptomycin, and L-glutamine). The medium can contain exogenously added ascorbic acid or a derivative thereof (e.g., ascorbic acid 2-phosphate: PAA). The medium can contain one or two or more selected from the group consisting of fatty acids or lipids, amino acids (e.g., nonessential amino acid), vitamins, growth factors (e.g., basic fibroblast growth factor: bFGF, platelet-derived growth factor AB, epidermal growth factor), nutritional factors (e.g., neurotrophic factors comprised of brain-derived neurotrophic factor: BDNF, glial cell line-derived neurotrophic factor: GDNF, neurotrophin 3: NT-3, neurotrophin 4/5, nerve growth factor, or the like), cytokines, antibiotics (e.g., penicillin and streptomycin), antioxidants, 2-mercaptoethanol, pyruvic acid, buffers, and inorganic salts.

The medium can contain exogenously added cytokines. Examples of cytokine include FLT3 ligands (FLT3L), interleukin-7 (IL-7), stem cell factors (SCF), thrombopoietin (TPO), IL-2, IL-3, IL-4, IL-6, IL-12, IL-15, IL-21, TNF-alpha, TGF-beta, interferon-gamma, interferon-lambda, TSLP, thymopentin, pleotrophin, and midkine. Any one of these cytokines can be used alone, or two or more can be used in combination.

In one embodiment of the present invention, the medium contains at least one selected from the group consisting of a basic fibroblast growth factor (bFGF), forskolin (FSK), and dorsomorphin (DM). In particular, the medium in the differentiation induction step preferably contains all of the basic fibroblast growth factor (bFGF), forskolin, and dorsomorphin.

The transcription factor introduction step and the differentiation induction step can be performed by two-dimensional culture (planar culture, or monolayer culture). As a culturing container, those having general shape such as flask, dish, or plate can be used, and a container to which a well is formed to accommodate cells can also be used. Culturing containers made of general materials such as glass, plastic or resin can be used. The surface of the culturing container may be untreated, or may be subjected to treatment related to adherence and proliferation features or the like of cells, or other treatment. For example, coating with poly-D-lysine and laminin or "iMatrix-511" (Nippi, Incorporated, human laminin 511-E8 fragment) can be said to be a preferable surface treatment in the present invention, from the viewpoint of the survival rate of the urine-derived cells (and fibroblasts) infected with viral vectors or the like. The size (area and volume) of the culturing container, and in case where the culturing container has a well, the size (diameter and depth) and the number of the well can be appropriately selected. As necessary, cells can be cultured while agitating the culturing container. The culturing environment is not particularly limited, and is preferably under conditions of about 5% CO₂, and about 37°C.

The application of the motor neurons obtained by the production method of the present invention is not particularly limited, and the motor neurons can be used as a disease model for diseases related to motor neurons such as amyotrophic lateral sclerosis (ALS), or can be used in a screening system for prophylactic agents or therapeutic agents; and may also be used in cell therapy. For example, the motor neurons produced from the urine-derived cells of a patient with a motor neuron disease can be used to evaluate drug efficacy (or toxicity) by culturing in a medium added with a candidate drug, and to search for substances that can act as a biomarker by analyzing culture supernatant. In addition, when using motor neurons produced from urine-derived cells of a plurality of patients, the motor neurons can be used for patient stratification or a study on companion diagnostics.

Further, the motor neurons obtained by the production method of the present invention can be used for treating or preventing a disease related to degeneration of motor neurons such as amyotrophic lateral sclerosis (ALS), myelopathic muscular atrophy, and spinobulbar muscular atrophy (SBMA).

The production method of the present invention can obtain, for example, the following effects.
(1) According to the production method of the present invention, obtained is a cell population of motoneurons that have high positive rate for each marker. Each marker includes at least one MN marker described herein (e.g., ISL1, LHX3, HB9, ChAT, SMI32, VAChT), and preferably further includes at least on neuronal marker (e.g., Tuj1, MAP2, NeuN, neurofilament, synapsin, PSD-95). In one preferred embodiment of the present invention, obtained is a cell population of motoneurons that have high positive rate for (X) at least one selected from the group consisting of ISL1, LHX3, HB9, ChAT, SMI32, and VAChT (MN markers) by the production method of the present invention. In one further preferred embodiment of the present invention, obtained is a cell population of motoneurons that have high positive rates (rates of cells positive for both X and Y) for (X) at least one selected from the group consisting of ISL1, LHX3, HB9, ChAT, SMI32, and VAChT (MN markers), and for (Y) at least one selected from the group consisting of Tuj1, MAP2, NeuN, neurofilament, synapsin, and PSD-95 by the production method of the present invention. In one particularly preferred embodiment of the present invention, obtained is a cell population of motoneurons that have high positive rates (rates of cells positive for both I and II) for (I) at least one selected from the group consisting of Tuj1 and ISL1, and for (II) at least one selected from the group consisting of HB9, ChAT and SMI32 by the production method of the present invention. The "positive rate" may vary depending on the type of marker, culturing conditions (culturing days or the like) or the like, and the positive rate can be set to, for example, 60% or more, 70% or more, 80% or more, 85% or more, 90% or more, or 95% or more. The positive rate can be measured by, for example, using fluorescent immunostaining images that target each marker, as shown in Examples.
(2) According to the production method of the present invention, obtained is a cell population of motoneurons that have high differentiation and maturation degrees. The differentiation and maturation degrees of the motoneurons can be evaluated by the length of nerve fiber, or the elongation rate of the length, and it can be said that the longer the nerve fiber, the higher the differentiation and maturation degrees. The length of the nerve fiber can be measured by, for example, using fluorescent immunostaining images that target each marker expressed by motoneurons, as shown in Examples.
(3) According to the production method of the present invention, obtained is a cell population of functional motoneurons. Examples of the functionality of the motoneurons include spontaneous firing, and formation of ganglion junction (NMJ) between muscle cells to control the movement of the muscles, and it can be evaluated as functional when the motoneurons have these properties, or are excellent in these properties. The spontaneous firing of the motoneurons can be confirmed or quantified by calcium imaging, that is, area under the curve (AUC) of calcium waveform, as shown in Examples, for example. Also, the control of the movement of muscle cells by motoneurons can be qualitatively or quantitatively evaluated by each marker expressed by motoneurons and skeletal muscle cells for NMJ formed when the motoneurons and skeletal muscle cells are co-cultured, or by fluorescent immunostaining images that target acetylcholine receptors, or can be evaluate by the contraction probability of the skeletal muscle cells.

In one aspect of the present invention, provided is a pharmaceutical composition (cell preparation) containing the motor neurons obtained by the production method of the present invention and the cells, for use in treating or preventing diseases as described above or the like.

Further, in another aspect of the present invention, provided is a method of administering a pharmaceutical composition (cell preparation) containing the motor neurons obtained by the production method of the present invention and the cells (cell therapy) for treating or preventing diseases as described above or the like.

The pharmaceutical composition (cell preparation) of the present invention can be formulated by a known pharmaceutical method, according to administration route or the like, and can be prepared as, for example, injections (intravenous injection, intravenous infusion, and the like), liquids, suspending agents and emulsions. In such formulation, pharmacologically acceptable carriers (media) and additives, specifically, sterile water, physiological saline, vegetable oil, solvent, base, emulsifier, suspending agent, surfactant, stabilizer, vehicle, preservative, diluent, isotonic agent, soothing agent, buffer, or solubilizer, can be appropriately used in combination. In addition, as preparation, Pharmacopeia compliant products and pharmaceutical additive-standard compliant products can be used. Further, other active ingredients (other agents, cells, or the like) according to therapeutic or prophylaxis purposes can be contained along with motor neurons.

The method of administering pharmaceutical composition (cell preparation) of the present invention is not particularly limited, and it is preferably parenteral administration such as intravenous, intraperitoneal, subcutaneous or intramuscular administration, or topical administration to an affected area, and more preferably intravenous administration or topical administration to an affected area. The dosage of the pharmaceutical composition (cell preparation) of the present invention can be appropriately adjusted according to age, body weight, symptoms and health condition of a subject, dosage form, mode of administration, or the like.

A product of the pharmaceutical composition (cell preparation) of the present invention may be labeled with a description to be used for treatment or prophylaxis of a disease or the like. For example, information telling that it is to be used for treatment or prophylaxis of a disease or the like can be described on a body, container, package, and the like of the product, or indication, package insert, printed matter for advertisements, other printed matters or the like.

The pharmaceutical composition (cell preparation) of the present invention has low toxicity (e.g., acute toxicity, chronic toxicity, genotoxicity, reproductive toxicity, cardiotoxicity, or carcinogenicity) and low side-effects, and it can be used as a prophylactic or therapeutic agents or a diagnostic agent for a disease as described above, to mammals (e.g., human, cattle, horse, dog, cat, pig, monkey, mouse, rat and rabbit).

The dosage of the pharmaceutical composition (cell preparation) of the present invention varies depending on the target subject, administration route, target disease, symptoms, or the like, and when orally or parenterally administering to an adult patient, for example, it is generally about 0.01 to 100 mg/kg body weight, preferably 0.1 to 50 mg/kg body weight, more preferably 0.5 to 20 mg/kg body weight as a single dose, and it is desirable to administer the amount 1 to 3 times per day.

The pharmaceutical composition (cell preparation) of the present invention can be used in combination with other drugs (hereinafter, abbreviated as concomitant drugs). By combining the pharmaceutical composition (cell preparation) of the present invention with concomitant drugs, the following excellent effects can be obtained:
(1) Comparing to the case where the pharmaceutical composition (cell preparation) of the present invention or concomitant drugs is administered alone, the dosage can be reduced;
(2) According to symptoms (mild, severe, or the like) of a patient, drugs to be combined with the pharmaceutical composition (cell preparation) of the present invention can be selected;
(3) By selecting concomitant drugs which mechanism of action is different from that of the pharmaceutical composition (cell preparation) of the present invention, the treatment period can be set long;
(4) By selecting concomitant drugs which mechanism of action is different from that of the pharmaceutical composition (cell preparation) of the present invention, it can be intended that the therapeutic effects be sustained; and
(5) By combining the pharmaceutical composition (cell preparation) of the present invention with concomitant drugs, synergistic effects can be obtained; and the like.

Hereinafter, use of the pharmaceutical composition (cell preparation) of the present invention combined with concomitant drugs is referred to as "concomitant agent of the present invention".

When using the concomitant agent of the present invention, administration timing of the pharmaceutical composition (cell preparation) of the present invention and the concomitant drugs is not limited, and the pharmaceutical composition (cell preparation) of the present invention or the pharmaceutical composition and the concomitant drugs or the pharmaceutical composition can be administered to a target subject simultaneously or with a time lag. The dosage of the concomitant drugs can be set according to the dosage clinically used, and can be appropriately selected depending on the subject to be administered, administration route, disease, combination, and the like.

The administration form of the concomitant agent of the present invention is not particularly limited, and any administration form can be selected as long as the pharmaceutical composition (cell preparation) of the present invention and the concomitant drugs are combined at the time of administration. Examples of the administration form include (1) administration of a single preparation obtained by formulating the pharmaceutical composition (cell preparation) of the present invention and the concomitant drugs at once, (2) simultaneous administration, through the same administration route, of two preparations obtained by separately formulating the pharmaceutical composition (cell preparation) of the present invention and the concomitant drugs, (3) administration, through the same administration route at different time points, of two preparations obtained by separately formulating the pharmaceutical composition (cell preparation) of the present invention and the concomitant drugs, (4) simultaneous administration, through different administration routes, of two preparations obtained by separately formulating the pharmaceutical composition (cell preparation) of the present invention and the concomitant drugs, and (5) administration, through different administration routes at different time points, of two preparations obtained by separately formulating the pharmaceutical composition (cell preparation) of the present invention and the concomitant drugs (e.g., administration in the order of the pharmaceutical composition (cell preparation) of the present invention and the concomitant drugs, or in the reverse order).

The dosage of the concomitant agent of the present invention can be appropriately selected based on the dosage clinically used. The mixing ratio of the pharmaceutical composition (cell preparation) of the present invention and the concomitant drugs can be appropriately selected according to the subject to be administered, administration route, target disease, symptoms, combinations or the like.

For example, the content of the pharmaceutical composition (cell preparation) of the present invention in the concomitant agent of the present invention varies depending on the form of the preparation; and generally, it is approximately about 0.01 to 100% by weight, preferably about 0.1 to 50% by weight, and more preferably about 0.5 to 20% by weight to the entire preparation.

The content of the concomitant drugs of the present invention in the concomitant agent of the present invention varies depending on the form of the preparation; and generally, it is approximately about 0.01 to 100% by weight, preferably about 0.1 to 50% by weight, and more preferably about 0.5 to 20% by weight to the entire preparation.

The content of the additives such as carrier in the concomitant agent of the present invention varies depending on the form of the preparation; and generally, it is approximately about 1 to 99.99% by weight, and preferably about 10 to 90% by weight to the entire preparation.

Also, it can be set to the similar content in a case of separately formulating the pharmaceutical composition (cell preparation) of the present invention and the concomitant drugs.

Examples of the concomitant drug include, but not limited to, the following. Therapeutic agents for narcolepsy (e.g., methylphenidate, amphetamine, pemoline, phenelzine, protriptyline, sodium oxybate, modafinil, and caffeine), anti-obesity agents (amphetamine, benzphetamine, bromocroptine, bupropion, diethylpropion, exenatide, fenfluramine, liothyronine, liraglutide, mazindol, methamphetamine, octreotide, octreotide, orlistat, phendimetrazine, phenmetrazine, phenmetrazine, phentermine, Qnexa (registered trademark), phenylpropanolamine, pramlintide, propylhexedrine, recombinant leptin, sibutramine, topiramate, zimelidine, zonisamide, lorcaserin, and metformin), acetylcholinesterase inhibitors (e.g., donepezil, rivastigmine, galantamine, zanapezil, idebenone, and tacrine), anti-dementia agents (e.g., memantine), β-amyloid protein production, secretion, accumulation, aggregation and/or deposition inhibitors, β-secretase inhibitors (e.g., 6-(4-biphenylyl)methoxy-2-[2-(N,N-dimethylamino)ethyl]tetralin, 6-(4-biphenylyl)methoxy-2-(N,N-dimethylamino)methyltetralin, 6-(4-biphenylyl)methoxy-2-(N,N-dipropylamino)methyltetralin, 2-(N,N-dimethylamino)methyl-6-(4'-methoxybiphenyl-4-yl)methoxytetralin, 6-(4-biphenylyl)methoxy-2-[2-(N,N-diethylamino)ethyl]tetralin, 2-[2-(N,N-dimethylamino)ethyl]-6-(4'-methylbiphenyl-4-yl)methoxytetralin, 2-[2-(N,N-dimethylamino)ethyl]-6-(4'-methoxybiphenyl-4-yl)methoxytetralin, 6-(2',4'-dimethoxybiphenyl-4-yl)methoxy-2-[2-(N,N-dimethylamino)ethyl]tetralin, 6-[4-(1,3-benzodioxole-5-yl)phenyl]methoxy-2-[2-(N,N-dimethylamino)ethyl]tetralin, 6-(3',4'-dimethoxybiphenyl-4-yl)methoxy-2-[2-(N,N-dimethylamino)ethyl]tetralin, an optically active substance thereof, a salt and hydrate thereof, and OM99-2 (International Publication No. WO01/00663)), γ-secretase inhibitory agents (e.g., PTI-00703, ALZHEMED (NC-531), PPI-368 (JP 1999(H11)-514333 A), PPI-558 (JP 2001-500852 A), and SKF-74652 (Biochem. J. (1999), 340(1), 283-289)), β-amyloid vaccines, β-amyloid degrading enzymes, brain function enhancers (e.g., aniracetam, and nicergoline), Parkinson's disease therapeutic agents [(e.g., dopamine receptor agonists (e.g., L-dopa, bromocriptine, pergolide, talipexole, pramipexole, cabergoline, and amantadine), monoamine oxidase (MAO) inhibitors (e.g., deprenyl, selegiline, remacemide, and riluzole), anticholinergic agents (e.g., trihexyphenidyl, and biperiden), and COMT inhibitors (e.g., entacapone)], amyotrophic lateral sclerosis therapeutic agents (e.g., neurotrophic factor such as riluzole), therapeutic agents for abnormal behavior, wandering behavior and the like associated with the progression of dementia (e.g., sedative drug, and antianxiety drug), apoptosis inhibitors (e.g., CPI-1189, IDN-6556, and CEP-1347), nerve differentiation/regeneration promotors (e.g., leteprinim, xaliproden (SR-57746-A), SB-216763, Y-128, VX-853, prosaptide, 5, 6-dimethoxy-2-[2,2,4,6,7-pentamethyl-3-(4-methylphenyl)-2,3-dihydro-1-benzofuran-5-yl]isoindoline, 5,6-dimethoxy-2-[3-(4-isopropylphenyl)-2,2,4,6,7-pentamethyl-2,3-dihydro-1-benzofuran-5-yl]isoindoline, 6-[3-(4-isopropylphenyl)-2,2,4,6,7-pentamethyl-2,3-dihydro-1-benzofuran-5-ylJ-6,7-dihydro-5H-[1,3]dioxolo[4,5-f]isoindole, and an optically active substance, salt and hydrate thereof), nonsteroidal anti-inflammatory agents (meloxicam, tenoxicam, indomethacin, ibuprofen, celecoxib, rofecoxib, aspirin, and the like), steroidal agents (dexamethasone, hexestrol, cortisone acetate, and the like), disease-modifying antirheumatic drugs (DMARDs), anti-cytokine drugs (e.g., TNF inhibitor, and MAP kinase inhibitor), urinary incontinence/frequent urination therapeutic agents (e.g., flavoxate hydrochloride, oxybutynin hydrochloride, and propiverine hydrochloride), phosphodiesterase inhibitors (e.g., sildenafil (citrate)), dopamine agonists (e.g., apomorphine), antiarrhythmic drugs (e.g., mexiletine), sex hormones or a derivative thereof (e.g., progesterone, estradiol, and estradiol benzoate), osteoporosis therapeutic agents (e.g., alfacalcidol, calcitriol, elcatonin, salmon calcitonin, estriol, ipriflavone, pamidronate disodium, alendronate sodium hydrate, and incadronate disodium), parathyroid hormones (PTH), calcium receptor antagonists, insomnia therapeutic agents (e.g., benzodiazepine-based drug, nonbenzodiazepine drug, melatonin agonist, and orexin receptor antagonist), schizophrenia therapeutic agents (e.g., typical antipsychotic such as haloperidol; atypical antipsychotic such as clozapine, olanzapine, risperidone, and aripiprazole; agents acting on metabotropic glutamate receptor or ion channel-coupled glutamate receptor; and phosphodiesterase inhibitor), benzodiazepine-based drugs (chlordiazepoxide, diazepam, clorazebate potassium, lorazepam, clonazepam, alprazolam, and the like), L-type calcium channel blockers (pregabalin, and the like), tricyclic or tetracyclic antidepressants (imipramine hydrochloride, amitriptyline hydrochloride, desipramine hydrochloride, clomipramine hydrochloride, and the like), selective serotonin reuptake inhibitors (fluvoxamine maleate, floxetine hydrochloride, citalopram bromide, sertraline hydrochloride, paroxetine hydrochloride, escitalopram oxalate, and the like), serotonin-noradrenaline reuptake inhibitors (venlafaxine hydrochloride, duloxetine hydrochloride, desvenlafaxine hydrochloride, and the like), noradrenaline reuptake inhibitors (reboxetine mesylate, and the like), mirtazapine, trazodone hydrochloride, nefazodone hydrochloride, bupropion hydrochloride, setiptiline maleate, 5-HT1A agonists (buspirone hydrochloride, tandospirone citrate, osemozotan hydrochloride, and the like), 5-HT2A antagonists, 5-HT2A inverse agonists, 5-HT3 antagonists (cyamemazine, and the like), non-cardioselective β-blockers (propranolol hydrochloride, oxprenolol hydrochloride, and the like), histamine H1 antagonists (hydroxyzine hydrochloride, and the like), CRF antagonists, other antianxiety drugs (meprobamate, and the like), tachykinin antagonists (MK-869, saredutant, and the like), agents acting on metabotropic glutamate receptor, CCK antagonists, β3 adrenaline antagonists (amibegron hydrochloride, and the like), GAT-1 inhibitors (tiagabine hydrochloride, and the like), N-type calcium channel blockers, Type-2 carbonic anhydrase inhibitors, NMDA glycinesite agonists, NMDA antagonists (memantine, and the like), peripheral benzodiazepine receptor agonists, vasopressin antagonists, vasopressin V1b antagonists, vasopressin V1a antagonists, phosphodiesterase inhibitors, opioid antagonists, opioid agonists, uridine, nicotinic acid receptor agonists, thyroid hormones (T3, T4), TSH, TRH, MAO inhibitors (phenelzine sulfate, tranylcypromine sulfate, moclobemide, and the like), bipolar disorder therapeutic agents (lithium carbonate, sodium valproate, lamotrigine, riluzole, felbamate, and the like), cannabinoids CB1 antagonists (rimonabant, and the like), FAAH inhibitors, sodium channel blockers, anti-ADHD drugs (methylphenidate hydrochloride, methamphetamine hydrochloride, and the like), alcoholism therapeutic agents, autism therapeutic agents, chronic fatigue syndrome therapeutic agents, convulsions therapeutic agents, fibromyalgia therapeutic agents, headache therapeutic agents, smoking cessation therapeutic agents, myasthenia gravis therapeutic agents, cerebral infarction therapeutic agents, mania therapeutic agents, hypersomnia therapeutic agents, pain therapeutic agents, dysthymia therapeutic agents, autonomic imbalance therapeutic agents, male and female sexual dysfunction therapeutic agents, migraine therapeutic agents, pathological gambling therapeutic agents, restless leg syndrome therapeutic agents, substance dependence therapeutic agents, alcohol-related disease therapeutic agents, therapeutic agents for irritable bowel syndrome, therapeutic agents for dyslipidemia such as cholesterol-lowering drugs (statin series (pravastatin sodium, atorvastatin, simvastatin, rosuvastatin, and the like), fibrates (clofibrate, and the like), squalene synthesis inhibitors), abnormal behavior therapeutic agents or dementia-induced wanderlust suppressants (sedatives, anxiolytics, and the like), diabetes therapeutic agents, diabetic complications therapeutic agents, hypertensive therapeutic agents, hypotensive therapeutic agents, diuretics, chemotherapeutic agents, immunotherapy agents, antithrombotic agents, anticancer agents, and the like.

Two or more of the above concomitant drugs can be used in combination at an appropriate ratio.

Further, the pharmaceutical composition (cell preparation) of the present invention can be used in combination with biopharmaceuticals (e.g., antibody therapeutic, nucleic acid or nucleic acid derivative, aptamer drug, and vaccine preparation) when applying to respective diseases described above; and use in combination with a gene therapy or the like, and use in combination with a therapy in the psychiatry domain where no drugs are used are also possible.

Examples of the antibody therapeutic and vaccine preparation include a vaccine preparation against angiotensin II, a vaccine preparation against CETP, a CETP antibody, a TNFα antibody or antibodies against other cytokines, amyloid β vaccine preparation, a vaccine for type-I diabetes (e.g., DIAPEP-277 from Peptor Ltd.), anti-HIV antibody and HIV vaccine preparation, as well as antibodies or vaccine preparations against cytokines, renin-angiotensin system enzymes and products thereof, antibodies or vaccine preparations against enzymes and proteins involved in blood lipid metabolism, antibodies or vaccine against enzymes and proteins involved in coagulation and fibrinolytic system in blood, and antibodies or vaccine preparations against proteins involved in glucose metabolism and insulin resistance. In addition to these, combination use with biopharmaceuticals related to growth factors such as GH and IGF is also possible.

Examples of the gene therapy include a therapy using genes related to cytokines, renin-angiotensin system enzymes and products thereof, G proteins, G protein-coupled receptors and phosphoenzymes thereof, a therapy using DNA decoys such as NFκB decoys, a therapy using antisense, a therapy using genes related to enzymes and proteins involved in blood lipid metabolism (e.g., genes related to metabolism, excretion, and absorption of cholesterol, triglyceride, HDL-cholesterol or blood phospholipid), a therapy using genes related to enzymes and proteins (e.g., growth factors such as HGF, VEGF) involved in angiogenic therapies for peripheral vascular occlusive disease and the like, a therapy using genes related to proteins involved in glucose metabolism and insulin resistance, and antisense against cytokines such as TNF.

Examples of the therapy in the psychiatry domain where no drugs are used include psychotherapies such as a modified electroconvulsive therapy, a deep brain stimulation therapy, a repetitive transcranial magnetic stimulation therapy, and a cognitive behavior therapy.

In addition, the pharmaceutical composition (cell preparation) of the present invention can be used in combination with various organ regeneration methods such as heart regeneration, kidney regeneration, pancreatic regeneration and blood vessel regeneration, cell transplantation therapies using bone marrow cells (bone marrow mononuclear cells and bone marrow stem cells), and artificial organs using tissue engineering (e.g., artificial blood vessels, and cardiomyocyte sheets)

As used herein and in the claims, unless otherwise required by context, words in the singular shall be deemed to include the plural and vice versa. Accordingly, articles in the singular (e.g., "a", "an", "the" and the like in English) should be understood to also include their plural concepts, unless otherwise specified.

### Examples

### <Materials and Methods>

The present invention will be described in more detail using Examples described below; however, the present invention is not limited to these. First, materials used in the following Examples and basic experimental techniques will be described.

### <Human Urine-Derived Cells (UDCs)>

In the following Examples, unless otherwise noted, used were 3 strains (3805-8505 derived from healthy individuals, and 3805-8506 and 3805-8507 derived from sporadic amyotrophic lateral sclerosis patients) obtained from REPROCELL Inc., Japan, and 5 strains derived from healthy individuals (UDC280, UDC283, UDC304, UDC305, and UDC306) obtained from Evercyte GmbH, Austria, as human urine-derived cells (UDCs).

### <Human Fibroblasts>

In the following Examples, unless otherwise noted, as fibroblasts derived from healthy individuals, used were catalog Nos. C-12302 obtained from PromoCell GmbH, Germany and 106-05n obtained from Cell Applications, Inc., USA.

### <Mouse Myoblast Cell Line C2C12>

In the following Examples, unless otherwise noted, the mouse myoblast cell line C2C12 (catalog No. CRL-1772) obtained from American Type Culture Collection (ATCC) was differentiated by a method described later to use as skeletal muscle cells.

### <Exogenous Nucleic Acids>

In the following Examples, the following human or mouse gene sequences were used as exogenous genes. Human NGN2 (silent mutation from NM_024019.4 with 1 base substitution), mouse Sox11 (NM_009234.6), human ISL1 (silent mutation from NM_002202.3 with 1 base substitution), and human LHX3 (NM_014564.5).

### <Lentiviral Vector Construction and Virus Packaging>

At VectorBuilder Inc., artificial synthetic genes obtained by connecting exogenous nucleic acids (the stop codon of the first gene was deleted) with a cleavage sequence T2A were incorporated into a virus vector pLV with a drug resistance gene to construct each of lentiviral vectors pLV[Exp]-Neo-CMV>hNGN2-T2A-mSox11 and pLV[Exp]-Puro-CMV>hISL1-T2A-hLHX3. The virus packaging was performed at VectorBuilder Inc., and the obtained virus solution was stored at -80°C. In the viral vectors, human NGN2 and mouse Sox11, and human ISL1 and human LHX3 are each bicistronically expressed under the CMV promoter.

### <Adenoviral Vector Construction and Virus Packaging>

At VectorBuilder Inc., artificial synthetic genes obtained by connecting exogenous nucleic acids (the stop codon of the first gene was deleted) with a cleavage sequence T2A were incorporated into a virus vector pAV to construct each of adenoviral vectors pAV[Exp]-CMV>hNGN2-T2A-mSox11 and pAV[Exp]-CMV>hISL1-T2A-hLHX3. The virus packaging was performed at VectorBuilder Inc., and the obtained virus solution was stored at -80°C. In the viral vectors, human NGN2 and mouse Sox11, and human ISL1 and human LHX3 are each bicistronically expressed under the CMV promoter.

### <Technique 1: Culture of Fibroblasts and Introduction of

### Exogenous Nucleic Acid Using Viral Vector>

Fibroblasts were cultured and maintained using a MEM medium containing FBS (15%, GIBCO), GlutaMAX (1%, GIBCO), and penicillin and streptomycin (1%, GIBCO). The fibroblasts were detached and isolated into single cells by treatment with 0.05% trypsin-EDTA (GIBCO), and seeded in a 96-well multiwell plate at a cell density of 10,000 cells/well. On the following day, the medium was replaced with a medium containing 2 viruses (in the case of lentivirus, 6 µm/mL polybrene was added thereto) to infect the cells with the viruses, leading to express 4 exogenous genes. On the day after virus infection, the medium was replaced with the medium for the fibroblast (described above) (Figure 1).

### <Technique 2: Culture of UDCs and Introduction of Exogenous Nucleic Acid Using Viral Vector>

UDCs from REPROCELL Inc. were cultured on a plastic plate coated with gelatin (Merck-Millipore) using a medium obtained by REPROCELL Inc. UDCs from Evercyte GmbH were cultured on a plastic plate coated with gelatin using a medium obtained by mixing Renal Epithelial Cell Growth Medium Bullet Kit (REGM, Lonza Group Ltd.) with a high glucose DMEM medium (GIBCO) containing FBS (30%, Corning Incorporated), GlutaMAX (1%, GIBCO), nonessential amino acids (1%, GIBCO), basic fibroblast growth factors AB (5 ng/mL, PeproTech, Inc.), platelet-derived growth factor AB (5 ng/mL, PeproTech, Inc.), epidermal growth factor (5 ng/mL, PeproTech, Inc.), and penicillin and streptomycin (2%, GIBCO) at a volume ratio of 1:1. The UDCs were detached and isolated into single cells by treatment with 0.05% or 0.25% of trypsin-EDTA, and seeded in a 96-well multiwell plate at a cell density of 10,000 cells/well. On the following day, the medium was replaced with a medium containing 2 viruses (in the case of lentivirus, 6 µg/mL polybrene was added thereto) to infect the cells with the viruses, leading to express 4 exogenous genes. On the day after virus infection, the medium was replaced with the medium for the UDCs (described above) (Figure 1).

### <Technique 3: Culture of C2C12 and Differentiation into Skeletal Muscle Cells>

C2C12 was cultured and maintained using a high glucose DMEM medium (FUJIFILM Wako Pure Chemical Corporation) containing FBS (10%, GIBCO), and penicillin and streptomycin (1%). After that, the medium was replaced with a high glucose DMEM medium containing FBS or horse serum (2%, GIBCO), insulin (1 µM, FUJIFILM Wako Pure Chemical Corporation), and penicillin and streptomycin (1%) to differentiate C2C12 into skeletal muscle cells.

### <Technique 3: Differentiation Induction into Motor Neurons After Introduction of Exogenous Nucleic Acids and Co-culture with Skeletal Muscle Cells>

On 2 days after virus infection, the medium was completely replaced with a neuro differentiation medium. As neuro differentiation media, a mixed medium of a DMEM/F12 medium (FUJIFILM Wako Pure Chemical Corporation) to which N2 supplement (0.8%, FUJIFILM Wako Pure Chemical Corporation), B27 supplement (0.8%, GIBCO), forskolin (10 µM, FUJIFILM Wako Pure Chemical Corporation), dorsomorphin (1 µM, FUJIFILM Wako Pure Chemical Corporation), basic fibroblast growth factors (basic FGF) (10 ng/mL, FUJIFILM Wako Pure Chemical Corporation), penicillin and streptomycin (1%) were added, and a Neurobasal medium (GIBCO) (at a mixed volume ratio of 2:1) was used until Day 14 of viral infection, and a mixed medium of a DMEM/F12 medium to which N2 supplement (0.8%), B27 supplement (0.8%), forskolin (5 µM), brain-derived neurotrophic factors (BDNF) (10 ng/mL, PeproTech, Inc.), glial cell line-derived neurotrophic factors (GDNF) (10 ng/mL, FUJIFILM Wako Pure Chemical Corporation) and neurotrophin 3 (NT-3) (10 ng/mL, FUJIFILM Wako Pure Chemical Corporation), penicillin and streptomycin (1%) were added, and a Neurobasal medium (at a mixed volume ratio of 2:1) was used after Day 14 of viral infection (Figure 1). The media were changed by half every 2 to 3 days. Co-culture with skeletal muscle cells were performed as follows: C2C12 cells that have been differentiated with 1% FBS or 2% horse serum and 1 µM insulin were detached by treatment with 0.05% trypsin-EDTA, and seeded on the motor neurons to culture for 7 days.

### <Technique 4: RNA Extraction, cDNA Synthesis and Quantitative PCR (qPCR)>

RNA was extracted with RNeasy Mini kit (QIAGEN N.V.) according to the instruction. cDNA was synthesized using SuperScript VILO cDNA Synthesis Kit (Invitrogen). qPCR was conducted by 7900HT Fast Real-Time PCR System (Applied Biosystems) using TaqMan Fast Advanced Master Mix (Invitrogen). TaqMan Gene Expression Assays were all obtained by Applied Biosystems (Table 1). The expression level was represented as a relative expression level (%) with respect to GAPDH.

**[Table 1]**

| TaqMan Gene Expression Assay | |
|---|---|
| Gene name | Assay ID |
| GAPDH | 4325792 |
| ChAT | Hs00758143_m1 |
| MNX1 (HB9) | Hs00907365_m1 |

### <Technique 5: Immunocytochemistry and Image Analysis>

Cells were fixed with paraformaldehyde (4%, FUJIFILM Wako Pure Chemical Corporation) for total of 30 minutes, and then the cells were membrane-permeabilized with Triton X-100 (0.1%, MP Biomedicals Inc.) and blocked with Protein-free T20 (TBS) Blocking Buffer (Pierce). After primary antibody was reacted at room temperature for an hour, washed with DPBS (FUJIFILM Wako Pure Chemical Corporation), and then the secondary antibody was reacted at room temperature for an hour. After washed with DPBS, nuclear staining was performed with a nuclear staining reagent. Fluorescent images were automatically obtained using CV7000 or CV8000 (Yokogawa Electric Corporation), and the ratio of cells expressing motoneuron markers, the length of the nerve fiber, and the number of α-bungarotoxin positive clusters were calculated by image analysis using CV7000 analyzing aid software or CellPath finder (Yokogawa Electric Corporation). As the primary antibody, the following was used. Tuj1 (R&D Systems, Inc., MAB 11995, 1:1000), Tuj1 (Cell Signaling Technology, Inc., 5568S, 1:200), HB9 (Abcam Limited, ab221884, 1:100), ISL1/2 (Developmental Studies Hybridoma Bank, 39.4D5-c, 1:100), ChAT (Millipore, AB14P, 1:100), SMI32 (BioLegend, Inc., 801701, 1:300), MyHC (R&D Systems, Inc., MAB4470, 1:1000), and SYP (Synaptic Systems GmbH, 101004, 1:1000). Alexa Fluor 647-labeled α-bungarotoxin (Invitrogen, B35450, 1:500) was used to detect acetylcholine receptors.

### <Technique 6: Calcium Imaging and Skeletal Muscle Contraction>

Cells were stained with a calcium indicator Cal-520AM (AAT Bioquest, Inc., 21130) and time lapse imaging was performed with CV8000 for 30 seconds. Fluorescent values were quantified by image analysis using CellPath finder, and waveform analysis was performed using Spotfire-based Wave Finder (TIBCO Software Inc.) to calculate the area under the curve (AUC). The contraction probability of skeletal muscle was calculated based on bright-field time-lapse images obtained by CV8000.

### <Comparative Study of Multiplicity of Infection (MOI) between Coating Agent and Virus>

First, in the induction differentiation method reported in Meng-Lu Liu et al. (2016) Cell Reports 14, 115-128 (Non Patent Literature 3) (in other words, a method for inducing direct differentiation of fibroblasts into motoneurons by introducing 4 transcription factors: human NGN2 and mouse Sox11, and human ISL1 and human LHX3, into human fibroblasts with lentivirus), for comparing coating agents with MOI when infecting with lentivirus, Matrigel (30 µg/cm², Corning Incorporated), iMatrix-511 (0.3 µg/cm², Nippi, Incorporated), and poly-D-lysine (PDL; 8 µg/cm² concentration, Sigma Aldrich Co. LLC)/laminin (10 µg/mL, Trevigen, Inc.) were used as the coating agent in Technique 1 above to infect human fibroblasts (106-05n) with lentivirus at multiple MOIs (20 to 100). According to Technique 3 above, the medium was switched to a neuro differentiation medium, and bright field images were obtained using CellVoyager CV7000 (Yokogawa Electric Corporation) on Days 7 and 14 after lentivirus infection (Figures 2A and 2B). Nerve-like cells were observed in any coatings and MOIs; however, the number of the remaining cells decreased as the days of differentiation increased at MOI = 100 (Figures 2A and 2B). In Matrigel, in particular, the number of the remaining cells decreased as MOI and the days of differentiation increased, and therefore, further comparative study was conducted focusing on iMatrix-511 and PDL/laminin. Here, MOI was fixed at 30. In the same manner as in the above-described method, immunostaining was performed with Tuj1 on Day 7 after lentivirus infection, and it was found that iMatrix-511 had higher positive rate for Tuj1 than PDL/laminin (Figure 2C). The above results suggested that iMatrix-511 obtains motoneurons the most efficiently, and therefore, iMatrix-511 was used as the coating agent in Examples, and MOI at the lentivirus infection was set to 30.

### [Example 1] Differentiation of Human UDCs and Human Fibroblasts into Motoneurons by Lentiviral Vector

It was examined whether UDCs could be differentiated into motoneurons by introduction of the above 4 transcription factors by lentivirus. First, when the gene expression of HB9 and ChAT, which are motoneuron markers, was observed by qPCR, it was confirmed that the expression increased after differentiation (Days 7 and 14) from before differentiation (Day 0) on both cases (Figure 3). Also, the results of immunostaining and its quantitative analysis indicated that the ratio of cells positive for Tuj1 and HB9, cells positive for ISL1 and ChAT, that were exogenously expressed, and cells positive for Tuj1 and SMI32 (motoneuron marker) increased from before differentiation (Figure 4). When comparing to fibroblasts, the UDCs generally had higher positive rate for each marker.

### [Example 2] Differentiation of Human UDCs and Human Fibroblasts into Motoneurons by Adenoviral Vector

Since the fact that fibroblasts could be induced to differentiate into motoneurons by using adenovirus had been obtained, for UDCs as well, it was examined whether UDCs could be differentiated into motoneurons by introduction of the 4 transcription factors by adenovirus. In the same manner as in Example 1, the gene expression of HB9 and ChAT was first observed by qPCR. As the result, it was revealed that not only the expression significantly increased after differentiation (Days 7 and 14) from before differentiation (Day 0) on both cases (Figure 5), but also the expression of both marker genes greatly increased by adenovirus (Figure 5), compared to the expression induction by lentivirus (Figure 3). Also, the results of immunostaining and its quantitative analysis indicated that the ratio of cells positive for Tuj1 and HB9, cells positive for ISL1 and ChAT, that were exogenously expressed, and cells positive for Tuj1 and SMI32 increased from before differentiation (Figure 6), as well as that the UDCs had higher positive rate for each marker, compared to the fibroblasts. Further, the positive rate for every marker was also higher compared to the differentiation induction by lentivirus (Figure 4). For SMI32, in particular, the degree of the expression induction when using the fibroblasts was small by both lentivirus and adenovirus; however, SMI32 was significantly induced to express by the UDCs, and its positive rate was high. In addition, when measuring the length of the nerve fiber stained by Tuj1 or SMI32 over time, it was confirmed that the length of the nerve fiber from the UDCs extends, as the days of differentiation increased, more than that from the fibroblasts (Figure 7). From the above, it was suggested that the differentiation and maturation degrees of the differentiation-induced motoneurons advanced more from the UDCs than those from the fibroblasts.

### [Example 3] Study on Functionality of Motoneurons Differentiated from Human UDCs by Adenoviral Vector

To study the functionality as motoneurons, first, it was examined by calcium imaging whether the motoneurons induced to differentiate from the UDCs spontaneously fires. As a result, spontaneous firing was observed at least from 14 days after differentiation (Figure 8A). The result of calcium waveform analysis revealed that the area under the curve (AUC) of the calcium waveform significantly increased after differentiation (Figure 8A). The role of the motoneurons in vivo is to innervate muscles and control its movement through the synaptic structure called neuromuscular junction. So next, it was examined whether neuromuscular junction was formed when skeletal muscle cells obtained by differentiating C2C12 and motoneurons induced to differentiate from UDCs were co-cultured, by using α-bungarotoxin, which specifically detects acetylcholine receptors. As a result, the number of clusters positive for α-bungarotoxin significantly increased when co-culturing with the motoneurons induced to differentiate from UDCs, compared to the case of C2C12 only or the case of co-culturing with UDCs themselves before differentiation (Figures 8B and 8C). Further, the probability of C2C12 contraction significantly increased when co-culturing with the motoneurons induced to differentiate from UDCs (Figure 8D). From the above, it was indicated that the motoneuron induced to differentiate from UDCs are functional as motoneurons.

### Industrial Applicability

According to the present invention, a cell population of motor neurons can be produced from urine-derived cells with less burden on a donor.

The present application is made based on Japanese Patent Application No. 2021-186927 filed in Japan, the content of which is incorporated herein in its entirety.

## Claims

1. A method for producing a motor neuron from a urine-derived cell, comprising
a step of introducing into the urine-derived cell a transcription factor for inducing differentiation into a motor neuron.

2. The method according to claim 1, wherein the transcription factor is introduced into the urine-derived cell by an adenoviral vector.

3. The method according to claim 1, further comprising a step of culturing the urine-derived cell into which the transcription factor has been introduced to obtain a cell positive for (X) at least one selected from the group consisting of ISL1, LHK3, HB9, ChAT, SMI32, and VAChT.

4. The method according to claim 1, further comprising a step of culturing the urine-derived cell into which the transcription factor has been introduced to obtain a cell positive for (X) at least one selected from the group consisting of ISL1, LHK3, HB9, ChAT, SMI32, and VAChT, and positive for (Y) at least one selected from the group consisting of Tuj1, MAP2, NeuN, neurofilament, synapsin, and PSD-95.

5. The method according to claim 1, further comprising a step of culturing the urine-derived cell into which the transcription factor has been introduced to obtain a cell positive for (I) at least one selected from the group consisting of Tuj1 and ISL1, and positive for (II) at least one selected from the group consisting of HB9, ChAT, and SMI32.

6. The method according to claim 5, wherein the step is to obtain a cell positive for at least SMI32.

7. The method according to claim 1, wherein the transcription factor comprises (i) at least one neurodifferentiation factor selected from the group consisting of NGN2, ASCL1, BRN2, MYT1L, NEUROD1, and miR-9/9*-124, and (ii) at least one motor neuron differentiation factor selected from the group consisting of ISL1, LHX3, and HB9.

8. The method according to claim 1, wherein the urine-derived cell into which the transcription factor has been introduced is cultured in a medium containing at least one selected from the group consisting of a basic fibroblast growth factor, forskolin, and dorsomorphin.

9. A cell population comprising a motor neuron obtained by the method according to claim 1.
